# EUROPEAN PATENT APPLICATION

(11) **EP 4 056 701 A1**
(43) Date of publication of application: **14.09.2022**
(21) Application number: 22154506.4
(22) Date of filing: 10.08.2017
(51) Int. Cl.: C12N 15/09

(54) **GENOME EDITING METHOD**

(30) Priority: 12.08.2016 JP 2016158562
(62) Divisional of application: 17839600.8
(71) Applicant: Nexuspiral Inc., Hyogo 6510096 (JP)
(72) Inventor: Maseda, Hideaki, Ikeda-shi, Osaka, 563-8577 (JP); Uwate, Maki, Tokushima-shi, Tokushima, 770-8501 (JP)
(74) Representative: Hoffmann Eitle

(57) **Abstract**

A technique for editing genome using a single strand polynucleotide consisting of: (i) (ia) a modified nucleotide sequence having a deletion of an arbitrary endogenous nucleotide sequence from an arbitrary nucleotide sequence in genomic DNA; (ib) a modified nucleotide sequence having an insertion of an arbitrary nucleotide sequence to an arbitrary nucleotide sequence in genomic DNA; or (ic) a modified nucleotide sequence having a substitution of an arbitrary endogenous nucleotide sequence with another nucleotide sequence in an arbitrary nucleotide sequence in genomic DNA; or (ii) a nucleotide sequence having 90% or more identity with the modified nucleotide sequence (except the same nucleotide sequence as the sense strand nucleotide sequence).

## Description

### Technical Field

The present invention relates to a method of editing genome.

### Background Art

A technique for editing genome called the CRISPR/Cas system has been developed and improved rapidly in recent years (Patent Literature 1). This technique makes it possible to cause DNA double strand cleavage in a particular sequence in the genome in a cell by using the combination of a guide RNA and a Cas protein. Since a mutation such as a random truncation or addition of DNA occurs frequently at the end of this DNA cleavage, gene disruption can be easily caused by the CRISPR/Cas system. Moreover, substitution, deletion, or the like of a nucleotide sequence in genomic DNA can also be caused by further combining a donor DNA containing a DNA sequence in the vicinity of the end of the DNA cleavage.

However, portions where a genomic DNA double strand cleavage can be caused by the CRISPR/Cas system are limited to the vicinity of the PAM sequence. Moreover, since random DNA truncation or addition occurs after DNA double strand cleavage, the change of the nucleotide sequence after the genome editing is unpredictable. Furthermore, since guide RNAs usually target a relatively short sequence with a length of about 20 nucleotides, such a guide RNA binds to sequences other than the intended target sequence to cause unintended genome editing (off-target effect).

### Citation List

### Patent Literature

Patent Literature 1: National Publication of International Patent Application No. 2016-500262
Patent Literature 2: International Publication No. WO 2015/115610

### Summary of Invention

### Technical Problem

An object of the present invention is to provide a simple method of editing genome. A preferable object of the present invention is to provide a simple method of editing genome having high flexibility to select portions to be modified by editing genome and lowered probability of unintended mutation other than the mutation of interest.

### Solution to Problem

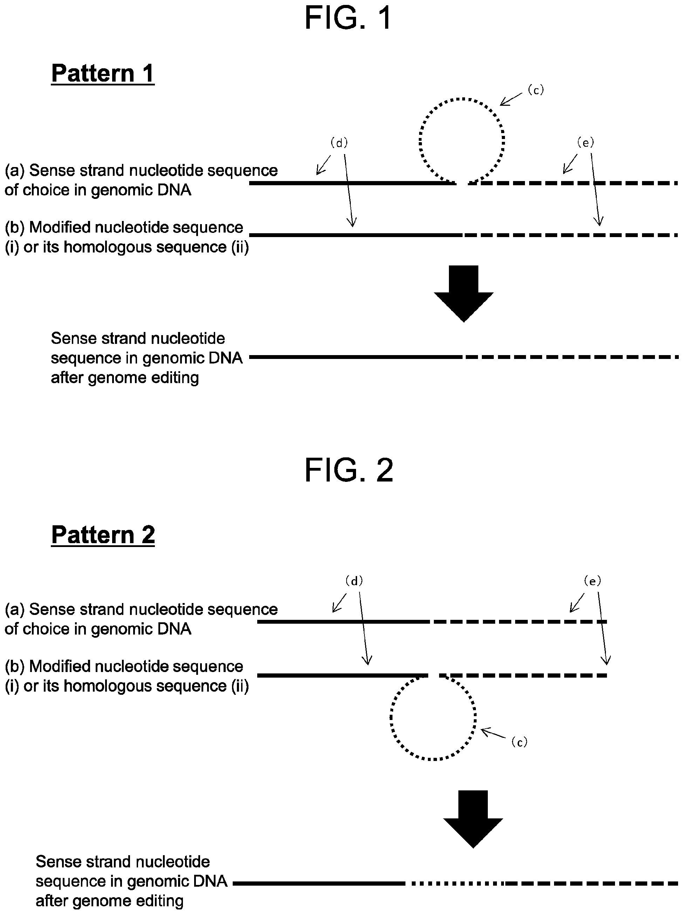

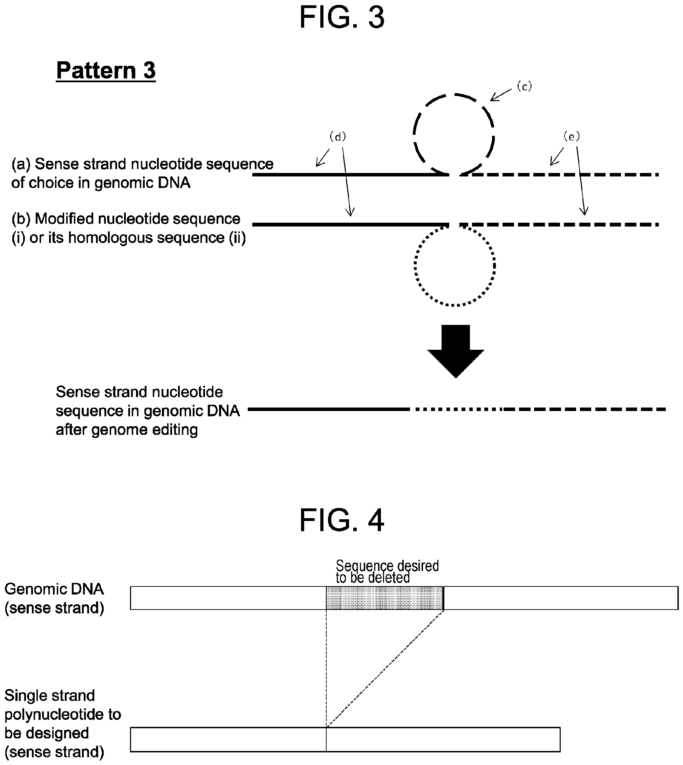

The present inventors have diligently studied to solve the aforementioned objects and surprisingly found as a result that only introducing into a cell a single strand polynucleotide consisting of:
(i) a modified nucleotide sequence from an arbitrary sense strand nucleotide sequence in genomic DNA by
   (ia) deletion of an arbitrary endogenous nucleotide sequence;
   (ib) insertion of an arbitrary nucleotide sequence; or
   (ic) substitution of an arbitrary endogenous nucleotide sequence with another nucleotide sequence results in the deletion in the genome when the arbitrary nucleotide sequence is deleted in the modified nucleotide sequence (Figure 1);
      results in the insertion in the genome when the arbitrary sequence is inserted in the modified nucleotide sequence; (Figure 2); and
      results in the substitution in the genome when the arbitrary nucleotide sequence is substituted in the modified nucleotide sequence (Figure 3). As a result of further study based on these findings, the present invention was achieved.

Accordingly, the present invention encompasses the following aspects:
Item 1.
   A method of editing genome, comprising the step of introducing into a cell or an organism at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA;
         wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
Item 2.
   The method of editing genome according to Item 1, comprising the step of synthesizing the at least one selected from the group consisting of the single strand polynucleotide and an expression vector thereof.
Item 3.
   The method of editing genome according to Item 1 or 2, further comprising the step of collecting the genome-edited cell or organism.
Item 4.
   The method of editing genome according to any of Items 1 to 3, further comprising the step of sorting the genome-edited cell or organism.
Item 5.
   The method of editing genome according to any of Items 1 to 4, not comprising any step of introducing at least one selected from the group consisting of a nuclease, a guide RNA, and an expression vector thereof.
Item 6.
   The method of editing genome according to any of Items 1 to 5, wherein the number of nucleotides in the modified nucleotide sequence is 80 to 1000.
Item 7.
   The method of editing genome according to any of Items 1 to 6, wherein the number of nucleotides in the modified nucleotide sequence is 90 to 300.
Item 8.
   The method of editing genome according to any of Items 1 to 7, wherein the number of nucleotides in the arbitrary nucleotide sequence to be deleted, inserted, or substituted is 1 to 920.
Item 9.
   The method of editing genome according to any of Items 1 to 8, wherein the ratio of the number of nucleotides in the arbitrary nucleotide sequence to be deleted, inserted, or substituted to the number of nucleotides in the modified nucleotide sequence is 10 or less.
Item 10.
   The method of editing genome according to any of Items 1 to 9, wherein the ratio of the number of nucleotides from the nucleotide adjacent 3' to the most 3' nucleotide of the arbitrary nucleotide sequence to be deleted, inserted, or substituted from the nucleotide at the 3' end of the sense strand nucleotide sequence to the number of nucleotides from the nucleotide at the 5' end of the sense strand nucleotide sequence to the nucleotide adjacent 5' to the most 5' nucleotide of the arbitrary nucleotide sequence to be deleted, inserted, or substituted is 0.2 to 5.
Item 11.
   The method of editing genome according to any of Items 1 to 10, wherein the single strand polynucleotide is a single strand DNA or single strand RNA.
Item 12.
   A composition for editing genome, comprising at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA;
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
Item 13.
   A kit for editing genome, comprising at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA;
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
Item 14.
   A method of producing a genome-edited cell or non-human organism, comprising the step of introducing into a cell or non-human organism at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA;
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
Item 15.
   A single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA;
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300.
Item 16.
   An expression vector comprising an expression cassette of the single strand polynucleotide according to Item 15.

### Advantageous Effects of Invention

According to the present invention, genome editing can readily be performed by only introducing a particular single strand polynucleotide derived from a sense strand of genomic DNA into a cell. This genome editing technique has high flexibility to select portions to be modified by editing and lowered probability of unintended mutation other than the mutation of interest. Moreover, off-target effect can be reduced by increasing the number of nucleotides in the single strand polynucleotide.

### Brief Description of Drawings

[Figure 1] Figure 1 illustrates the summary of a pattern of the method of editing genome according to the present invention (Pattern 1). The lines represent the nucleotide sequences of single strand polynucleotides. In each line, the left is the 5' side and the right is the 3' side. (a) represents an arbitrary sense strand nucleotide sequence in genomic DNA. (b) represents a modified nucleotide sequence (i) or its homologous sequence (ii). (c) represents the arbitrary nucleotide sequence deleted in the modified nucleotide sequence. (d) represents the nucleotide sequence from the nucleotide at the 5' end of the arbitrary sense strand nucleotide sequence in genomic DNA to the nucleotide adjacent 5' to the most 5' nucleotide of the nucleotide sequence to be deleted. (e) represents the nucleotide sequence from the nucleotide adjacent 3' to the most 3' nucleotide of the arbitrary nucleotide sequence to be deleted to the nucleotide at the 3' end of the sense strand nucleotide sequence.
[Figure 2] Figure 2 illustrates the summary of a pattern of the method of editing genome according to the present invention (Pattern 2). The lines represent the nucleotide sequences of single strand polynucleotides. In each line, the left is the 5' side and the right is the 3' side. (a) represents an arbitrary sense strand nucleotide sequence in genomic DNA. (b) represents a modified nucleotide sequence (i) or its homologous sequence (ii). (c) represents the arbitrary nucleotide sequence inserted in the modified nucleotide sequence. (d) represents the nucleotide sequence from the nucleotide at the 5' end of the arbitrary sense strand nucleotide sequence in genomic DNA to the nucleotide adjacent 5' to the most 5' nucleotide of the nucleotide sequence to be inserted. (e) represents the nucleotide sequence from the nucleotide adjacent 3' to the most 3' nucleotide of the arbitrary nucleotide sequence to be inserted to the nucleotide at the 3' end of the sense strand nucleotide sequence.
[Figure 3] Figure 3 illustrates the summary of a pattern of the method of editing genome according to the present invention (Pattern 3). The lines represent the nucleotide sequences of single strand polynucleotides. In each line, the left is the 5' side and the right is the 3' side. (a) represents an arbitrary sense strand nucleotide sequence in genomic DNA. (b) represents a modified nucleotide sequence (i) or its homologous sequence (ii). (c) represents the arbitrary nucleotide sequence substituted in the modified nucleotide sequence. (d) represents the nucleotide sequence from the nucleotide at the 5' end of the arbitrary sense strand nucleotide sequence in genomic DNA to the nucleotide adjacent 5' to the most 5' nucleotide of the nucleotide sequence to be substituted. (e) represents the nucleotide sequence from the nucleotide adjacent 3' to the most 3' nucleotide of the arbitrary nucleotide sequence to be substituted to the nucleotide at the 3' end of the sense strand nucleotide sequence.
[Figure 4] Figure 4 illustrates the summary of designing of a single strand polynucleotide which is a genome editing tool in the present invention when a desired sequence is desired to be deleted.
[Figure 5] Figure 5 illustrates the summary of designing of a single strand polynucleotide which is a genome editing tool in the present invention when a desired sequence is desired to be inserted.
[Figure 6] Figure 6 illustrates the summary of designing of a single strand polynucleotide which is a genome editing tool in the present invention when a desired sequence is desired to be substituted with another sequence.
[Figure 7] Figure 7 illustrates the correspondence relation between the sequences of single strand DNAs (Example 1) and genomic DNA. "-" indicates a nucleotide deleted in single strand DNAs and "}" indicates the sequence and the position inserted in genomic DNA sequence. "... - nucleotides ..." indicates that the number of nucleotides indicated by "-" are deleted.
[Figure 8] Figure 8 illustrates the coding sequence of a modified GFP (Example 6). This coding sequence is a sequence modified from the coding sequence of GFP by insertion of 8 nucleotides. The inserted 8 nucleotides are framed with a line.
[Figure 9] Figure 9 is a fluorescence observation image (Example 6).

### Description of Embodiments

As used herein, the expression "comprising" comprises the concepts of "comprising," "essentially consisting of," and "consisting of."

The "identity" of nucleotide sequences refers to the degree of amino acid sequence matching of 2 or more comparable amino acid sequences to one another. Therefore, if matching between two certain amino acid sequences is high, then the identity or similarity between the sequences is high. The level of identity of amino acid sequences is determined, for example, by using FASTA, a tool for sequence analysis, with default parameters. Alternatively, the level of identity may be determined by using BLAST, an algorithm by Karlin and Altschul (Karlin S, Altschul SF. "Methods for assessing the statistical significance of molecular sequence features by using general scoring schemes," Proc Natl Acad Sci USA. 87: 2264-2268 (1990), Karlin S, Altschul SF. "Applications and statistics for multiple high-scoring segments in molecular sequences.", Proc Natl Acad Sci USA. 90:5873-7 (1993)). A program called BLASTX, which is based on such an algorithm of BLAST has been developed. Specific procedures of these methods of analysis are known; see the web site of National Center of Biotechnology Information (NCBI)
(http://www.ncbi.nlm.nih.gov/).

The present invention relates to, in one aspect thereof, a composition for editing genome, a kit for editing genome, a method of editing genome, a method of producing a genome-edited cell or non-human organism, and the like, utilizing a single strand polynucleotide (herein, also referred to as a "single strand polynucleotide according to the present invention") consisting of:
(i)
   (ia) a modified nucleotide sequence having a deletion of an arbitrary endogenous nucleotide sequence from an arbitrary nucleotide sequence in genomic DNA;
   (ib) a modified nucleotide sequence having an insertion of an arbitrary nucleotide sequence to an arbitrary nucleotide sequence in genomic DNA; or
   (ic) a modified nucleotide sequence having a substitution of an arbitrary endogenous nucleotide sequence with another nucleotide sequence in an arbitrary nucleotide sequence in genomic DNA; or
(ii) a nucleotide sequence having 90% or more identity with the modified nucleotide sequence (except the same nucleotide sequence as the sense strand nucleotide sequence); and
   the single strand polynucleotide according to the present invention and an expression vector thereof (herein, also referred to as "the expression vector according to the present invention").

While not wishing to be bound by any limiting construction, the mechanism of the genome editing according to the present invention is considered to be as follows.

The present inventors have reported the presence of a law stating that in nucleotide sequences represented by the formula: X-Y-X-Y-X [wherein X and Y are different nucleotide sequences whose numbers of nucleotides are 1 or more] (herein, also referred to as "PODiR (Partially Overlapped Direct Repeat) sequences"), which are combined nucleotide sequences of the sequence X-Y-X and the sequence X-Y-X with an overlap of X, the non-overlapped sequence (X-Y) will be deleted in prokaryotes such as bacteria (Patent Literature 2). Moreover, the present inventors also have reported the presence of a law stating that PODiR sequences are generated by inserting nucleotide sequences represented by X-Y at a site adjacent to the 5' end of the sequence X-Y-X in the genome (Patent Literature 2). Furthermore, the present inventors have found that these laws are also conserved in eukaryotic cells.

During the analysis of the mechanism of this phenomenon (Phenomenon 1), the present inventors found a phenomenon in which expression of a protein encoded by DNA containing the PODiR sequence results in the production of mRNA in which the nucleotide sequence represented by X-Y in the PODiR sequence is deleted (Phenomenon 2).

Based on Phenomenon 1 and Phenomenon 2, the present inventors thought that the deletion of the nucleotide sequence represented by X-Y of the PODiR sequence in the genome (Phenomenon 1) may be caused by interaction (for example, hybridization) of the mRNA in which the nucleotide sequence represented by X-Y in the PODiR sequence is deleted (Phenomenon 2) that has been transcribed from the DNA containing the PODiR sequence in the genome, with DNA containing the PODiR sequence in the genome and by cleavage of the resulting excess portion (X-Y in genomic DNA) that cannot interact with the DNA by some kind of enzyme. Furthermore, the present inventors thought that the generation of the PODiR sequence by insertion of the nucleotide sequence represented by X-Y at a site adjacent to the 5' end of the sequence X-Y-X in the genome (Phenomenon 1) may be caused by interaction (for example, hybridization) of mRNA having the PODiR sequence with the DNA containing the sequence represented by X-Y-X (non-PODiR sequence) in the genome and insertion of the nucleotide sequence of the resulting excess portion (X-Y in mRNA) that cannot interact with the DNA into the genomic DNA during DNA replication.

Based on these thoughts, the present inventors have gotten an idea that artificial introduction of a single strand polynucleotide corresponding to the mRNA in Phenomenon 2, that is to say, a single strand polynucleotide consisting of a modified nucleotide sequence (portion (b) in Figure 1) from an arbitrary sense strand nucleotide sequence (portion (a) in Figure 1) in genomic DNA by deletion of an arbitrary nucleotide sequence (portion (c) in Figure 1) may result in interaction (for example, hybridization) of the polynucleotide with genomic DNA and cleavage of the resulting excess portion that cannot interact with the genomic DNA (the arbitrary nucleotide sequence in genomic DNA having deletion in the single strand polynucleotide: portion (c) in Figure 1) with some kind of enzyme (Figure 1). Furthermore, the present inventors have gotten an idea that artificial introduction of a single strand polynucleotide consisting of a modified nucleotide sequence (portion (b) in Figure 2) from an arbitrary sense strand nucleotide sequence (portion (a) in Figure 2) in genomic DNA by insertion of an arbitrary nucleotide sequence (portion (c) in Figure 2) may result in interaction (for example, hybridization) of the polynucleotide with genomic DNA and insertion of the resulting excess portion that cannot interact with the genomic DNA (the arbitrary nucleotide sequence inserted in the single strand polynucleotide: portion (c) in Figure 2) into the genomic DNA during DNA replication (Figure 2). Moreover, the present inventors thought that artificial introduction of a single strand polynucleotide consisting of a modified nucleotide sequence (portion (b) in Figure 3) from an arbitrary sense strand nucleotide sequence (portion (a) in Figure 3) in genomic DNA by substitution of an arbitrary nucleotide sequence (portion (c) in Figure 3) with another nucleotide sequence may result in interaction (for example, hybridization) of the polynucleotide with genomic DNA and reflection of the resulting excess portion that cannot interact with the genomic DNA (the portion to be present after substitution in the single strand polynucleotide) in the genomic DNA during DNA replication (Figure 3).

The genome editing according to the present invention is considered to be caused by such a mechanism since it has been demonstrated in Examples herein that this idea is correct.

According to the present invention, a desired mutation (deletion, insertion, or substitution) can be introduced into genomic DNA by designing a nucleotide sequence of single strand polynucleotide as described below.

Designing when a certain sequence in genomic DNA is desired to be deleted is illustrated in Figure 4. When a certain sequence is desired to be deleted in genomic DNA (the upper part in Figure 4), a single strand polynucleotide is designed such that, when it is hybridized with genomic DNA, the sequence desired to be deleted protrudes like a "swelling" (portion (c) in Figure 1) by being left out of the hybridization. More specifically, a single strand polynucleotide consisting of a sequence obtained by connecting the sequences (corresponding to portions (d) and (e) in Figure 1) on both sides of the sequence desired to be deleted in genomic DNA is designed as illustrated in the lower part in Figure 4.

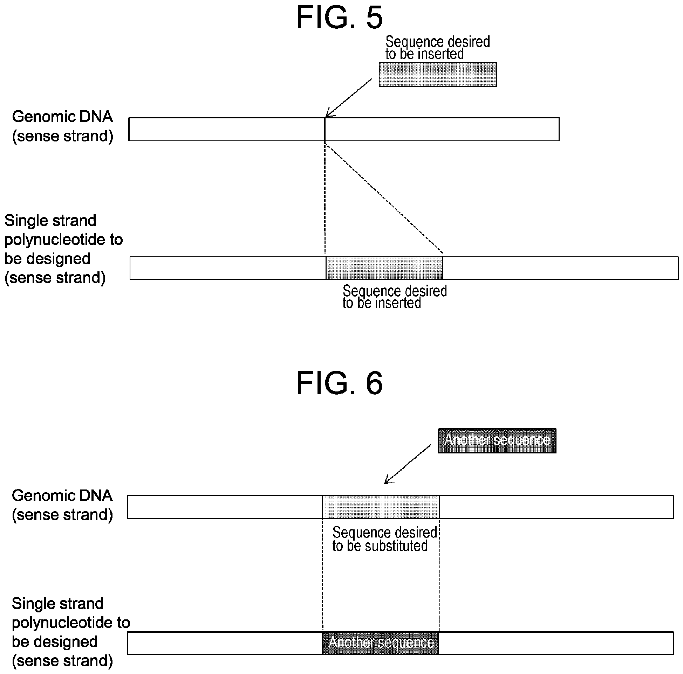

Moreover, designing when a certain sequence is desired to be inserted at a particular portion in genomic DNA is illustrated in Figure 5. When a certain sequence is desired to be inserted at a particular portion in genomic DNA (the upper part in Figure 5), a single strand polynucleotide is designed such that, when it is hybridized with genomic DNA, the sequence desired to be inserted protrudes like a "swelling" (portion (c) in Figure 2) by being left out of the hybridization. More specifically, a single strand polynucleotide consisting of a sequence having the sequences (corresponding to portions (d) and (e) in Figure 2) on both sides of the portion desired to have the insertion in genomic DNA and the sequence desired to be inserted flanked thereby is designed as illustrated in the lower part in Figure 5.

Furthermore, designing when a certain sequence in genomic DNA is desired to be substituted with another sequence is illustrated in Figure 6. When a certain sequence in genomic DNA is desired to be substituted with another sequence (the upper part in Figure 6), a single strand polynucleotide is designed such that, when it is hybridized with genomic DNA, a certain sequence in genomic DNA (the sequence desired to be substituted = the sequence to be substituted) and the other sequence (the substituting sequencing) protrude like "swelling" (portion (c) in Figure 3) by being left out of the hybridization. More specifically, a single strand polynucleotide consisting of a sequence having the sequences (corresponding to portions (d) and (e) in Figure 3) on both sides of the sequence to be substituted and the desired sequence (= the other sequence) flanked thereby is designed as illustrated in the lower part in Figure 6.

More specifically, a desired mutation (deletion, insertion, or substitution) can be introduced into genomic DNA by designing the nucleotide sequence of the single strand polynucleotide according to the present invention, as an example, as follows:
A) specifying (ia) nucleotides just before and after the sequence to be deleted, (ib) 2 consecutive nucleotides at the site of insertion (nucleotides just before and after the site), and (ic) nucleotides just before and after the sequence to be substituted on a sense strand sequence;
B) specifying a nucleotide(s) to be
   deleted/inserted/substituted (null in the case of deletion);
C) placing, upstream of the nucleotide specified in B) above, X nucleotides upstream of the nucleotide just before the sequence or site on the sense strand and downstream of the nucleotide identified in B) above, X nucleotides downstream of the nucleotide just before the sequence or site on the sense strand, respectively.

In one embodiment, the X nucleotides are the longer of at least 40 nucleotides and the 1/10 times of the number of nucleotides intended to be modified. The nucleotides to be modified are the largest number among 1. the number of nucleotides to be deleted, 2. the number of nucleotides to be inserted, 3. nucleotides to be removed/introduced by substitution.

Modification of the X nucleotides upstream of the nucleotide just before the sequence or site on the sense strand/the X nucleotides downstream of the nucleotide just before the sequence or site on the sense strand is not preferred since unintended genome editing occurs when modified.

### 1. Single strand polynucleotide

The single strand polynucleotide according to the present invention is a single strand polynucleotide consisting of:
(i)
   (ia) a modified nucleotide sequence having a deletion of an arbitrary endogenous nucleotide sequence from an arbitrary nucleotide sequence in genomic DNA;
   (ib) a modified nucleotide sequence having an insertion of an arbitrary nucleotide sequence to an arbitrary nucleotide sequence in genomic DNA; or
   (ic) a modified nucleotide sequence having a substitution of an arbitrary endogenous nucleotide sequence with another nucleotide sequence in an arbitrary nucleotide sequence in genomic DNA; or
(ii) a nucleotide sequence having 90% or more identity with the modified nucleotide sequence.

The "arbitrary sense strand nucleotide sequence in genomic DNA" (portion (a) in Figures 1 to 3) is an original nucleotide sequence of the nucleotide sequence of the single strand polynucleotide according to the present invention (the modified nucleotide sequence or homologous sequence thereof).

The "genomic DNA" is not particularly limited, as long as it is genomic DNA that the cell or organism to be modified by editing genome has.

The cells and organisms to be modified by editing genome are not particularly limited. According to the mechanism of the aforementioned genome editing according to the present invention, the genome editing according to the present invention may occur in any cells and organisms in which the phenomena of deletion and generation of the PODiR sequence (which have been found to occur not only in prokaryotic cells but also in eukaryotic cells) occur.

Examples of the organism to be modified by editing genome include a wide range of organisms, regardless of whether it is prokaryotes or eukaryotes. Examples of the prokaryotes include Gram negative bacteria (Pseudomonas aeruginosa, Escherichia coli, Serratia, Sphingomonas, Brucella, Neisseria gonorrhoeae, Burkholderia, dysentery, Salmonellae, Acinetobacter, Vibrio cholerae, Klebsiella pneumoniae, Legionella pneumophila, Helicobacter pylori, and Campylobacter), Gram positive bacteria (Mycobacterium tuberculosis, Mycoplasma, Staphylococcus aureus, Actinomycete, Bacillus subtilis, and Bacillus anthracis). Of these, preferable examples include the Gram negative bacteria, more preferable examples include Pseudomonas aeruginosa, Escherichia coli, Serratia, and Sphingomonas, and further preferable examples include Pseudomonas aeruginosa, and Escherichia coli. Examples of the eukaryote include animals such as mammalian animals such as humans, monkeys, mice, rats, dogs, cats, and rabbits; birds such as chickens; reptiles such as tortoises and crocodiles; amphibian animals such as Xenopus; fish animals such as zebra fishes, Olyzias latipes, and Takifugu rubripes; chordates such as ascidians; and arthropods such as Drosophila and silk worm: plants such as Arabidopsis, rice, wheat, and tobacco: fungi such as yeasts, Neurospora, Tricholoma matsutake, Lentinula edodes, Flammulina velutipes, Lyophyllum shimeji, Pholiota nameko, and Pleurotus eryngii. In one embodiment, the organism to be modified by editing genome is an "organism" including humans. Moreover, in another one embodiment, the organism to be modified by editing genome is a "non-human organism" other than humans.

Examples of the cell to be modified by editing genome include cells derived from various tissues or of various properties, for example, animal cells such as blood cells, hematopoietic stem cells, progenitor cells, gametes (sperm and ova), fertilized eggs, fibroblasts, epithelial cells, vascular endothelial cells, neural cells, hepatocytes, keratinocytes, muscular cells, epidermal cells, endocrine cells, ES cells, iPS cells, tissue stem cells, and cancer cells; and plant cells such as parenchyma cells and collenchyma cells.

The "sense strand" is the DNA strand that does not serve as template in transcription among the DNA double strands in the region to be transcribed into RNA in genomic DNA. The sense strand is preferably the sense strand of a coding region. The "coding regions" are not limited to coding regions for proteins, but include coding regions for RNAs (for example, miRNA, piRNA, tRNA, rRNA, snRNA, gRNA, SRP RNA, pRNA, tncRNA, sbRNA, snlRNA, SLRNA) not encoding any protein.

The "arbitrary sense strand nucleotide sequence" is not particularly limited as long as it is an arbitrary sequence in a sense strand nucleotide sequence. The number of nucleotides in the arbitrary sense strand nucleotide sequence is not particularly limited, and is for example, about 20 to 12000, preferably 20 to 3000, more preferably 40 to 2000, further more preferably 60 to 1000, further more preferably 80 to 600, further more preferably 90 to 300, and particularly preferably 95 to 200.

The "modified nucleotide sequence having deletion of an arbitrary endogenous nucleotide sequence from an arbitrary sense strand nucleotide sequence in genomic DNA" (portion (b) in Figure 1) is a nucleotide sequence modified from the arbitrary sense strand nucleotide sequence in genomic DNA by deletion of the arbitrary nucleotide sequence in the sense strand nucleotide sequence. The "deletion" here is removal of an inner portion of the arbitrary sense strand nucleotide sequence in genomic DNA, but not removal of a portion containing an end of the sense strand nucleotide sequence. Thus, in the modified nucleotide sequence, sequences derived from the original sense strand nucleotide sequence of the modification are remained at both the 5' and 3' ends. In other words, the modified nucleotide sequence has sequences derived from the original sense strand nucleotide sequence of the modification at both the 5' and 3' ends. The aforementioned "arbitrary nucleotide sequence" is one consecutive nucleotide sequence.

The number of nucleotides in the "modified nucleotide sequence" in the case of such a pattern in Figure 1 is not particularly limited, and is for example, about 20 to 2000, preferably 40 to 2000, more preferably 60 to 1000, further preferably 80 to 1000, further more preferably 80 to 600, further more preferably 90 to 300, and particularly preferably 95 to 200.

The "modified nucleotide sequence having insertion of an arbitrary nucleotide sequence to an arbitrary sense strand nucleotide sequence in genomic DNA" (portion (b) in Figure 2) is a nucleotide sequence modified from the arbitrary sense strand nucleotide sequence in genomic DNA by insertion of the arbitrary nucleotide sequence. The "insertion" here is insertion of an arbitrary nucleotide sequence into an inner portion of the arbitrary sense strand nucleotide sequence in genomic DNA, but not addition of an arbitrary nucleotide sequence at the end of the sense strand nucleotide sequence. Thus, in the modified nucleotide sequence, sequences derived from the original sense strand nucleotide sequence of the modification are remained at both the 5' and 3' ends. In other words, the modified nucleotide sequence has sequences derived from the original sense strand nucleotide sequence of the modification at both the 5' and 3' ends. The aforementioned "arbitrary nucleotide sequence" is one consecutive nucleotide sequence.

The number of nucleotides in the "modified nucleotide sequence" in the case of such a pattern in Figure 2 is not particularly limited, and is for example, about 20 to 12000, preferably 20 to 2000, more preferably 40 to 2000, further preferably 60 to 1000, further more preferably 80 to 1000, further more preferably 80 to 600, further more preferably 90 to 300, and particularly preferably 95 to 200.

The "modified nucleotide sequence having substitution of an arbitrary endogenous nucleotide sequence with another nucleotide sequence with another nucleotide sequence in an arbitrary sense strand nucleotide sequence in genomic DNA " (portion (b) in Figure 3) is a nucleotide sequence modified from the arbitrary sense strand nucleotide sequence in genomic DNA by substitution of the arbitrary nucleotide sequence in the sense strand nucleotide sequence with another nucleotide sequence. The "substitution" here is substitution of an inner portion of the arbitrary sense strand nucleotide sequence in genomic DNA, but not substitution of a portion containing an end of the sense strand nucleotide sequence. Thus, in the modified nucleotide sequence, sequences derived from the original sense strand nucleotide sequence of the modification are remained at both the 5' and 3' ends. In other words, the modified nucleotide sequence has sequences derived from the original sense strand nucleotide sequence of the modification at both the 5' and 3' ends. Moreover, "another nucleotide sequence" is not particularly limited as long as it is a nucleotide sequence different from the nucleotide sequence to be substituted, and for example, a nucleotide sequence having 0 to 50%, 0 to 20%, 0 to 10%, or 0 to 5% identity with the nucleotide sequence to be substituted. The aforementioned "arbitrary nucleotide sequence" is one consecutive nucleotide sequence.

The number of nucleotides in the "modified nucleotide sequence" in the case of such a pattern in Figure 3 is not particularly limited, and is for example, about 20 to 12000, preferably 20 to 2000, more preferably 40 to 2000, further preferably 60 to 1000, further more preferably 80 to 1000, further more preferably 80 to 600, further more preferably 90 to 300, and particularly preferably 95 to 200.

The "arbitrary nucleotide sequence" to be deleted, inserted, or substituted (portion (c) in Figures 1 to 3) is a nucleotide sequence to be deleted, inserted, or substituted in the genome by editing genome and the sequence is not particularly limited. The number of nucleotides in the "arbitrary nucleotide sequence" to be deleted, inserted, or substituted is not particularly limited, and is for example, 1 to 10000, preferably 1 to 1000, more preferably 1 to 920, further preferably 2 to 500, further more preferably 4 to 200, further more preferably 4 to 100, further more preferably 5 to 50, further more preferably 6 to 20, further more preferably 7 to 12, and further more preferably 8 to 9.

The ratio of the "arbitrary nucleotide sequence" to be deleted, inserted, or substituted (portion (c) in Figures 1 to 3) to the number of nucleotides in the "modified nucleotide sequence" (portion (b) in Figures 1 to 3) (i.e., the number of nucleotides in portion (c)/the number of nucleotides in portion (b)) is not particularly limited, and is for example, 500 or less, preferably 100 or less, more preferably 10 or less, further preferably 5 or less, further more preferably 2 or less, and further more preferably 1 or less. The lower limit of the ratio is not particularly limited, and is for example, 0.01, 0.02, 0.04, or the like.

The number of nucleotides ((d) in Figures 1 to 3) from the nucleotide at the 5' end of the "arbitrary sense strand nucleotide sequence in genomic DNA" (portion (a) in Figures 1 to 3) to the nucleotide adjacent 5' to the most 5' nucleotide of the "arbitrary nucleotide sequence" to be deleted, inserted, or substituted (portion (c) in Figures 1 to 3) is not particularly limited, and is for example, about 10 to 1000, preferably 20 to 1000, more preferably 30 to 500, further preferably 40 to 300, further more preferably 40 to 100, and particularly preferably 40 to 70.

The number of nucleotides ((e) in Figures 1 to 3) from the nucleotide adjacent 3' to the most 3' nucleotide of the "arbitrary nucleotide sequence" to be deleted, inserted, or substituted (portion (c) in Figures 1 to 3) to the nucleotide at the 3' end of the "arbitrary sense strand nucleotide sequence in genomic DNA" (portion (a) in Figures 1 to 3) is not particularly limited, and is for example, about 10 to 1000, preferably 20 to 1000, more preferably 30 to 500, further preferably 40 to 300, further more preferably 40 to 100, and particularly preferably 40 to 70.

In a preferred aspect of the present invention, both or at least one of the number of nucleotides ((d) in Figures 1 to 3) from the nucleotide at the 5' end of the "arbitrary sense strand nucleotide sequence in genomic DNA" (portion (a) in Figures 1 to 3) to the nucleotide adjacent 5' to the most 5' nucleotide of the "arbitrary nucleotide sequence" to be deleted, inserted, or substituted (portion (c) in Figures 1 to 3) and
the number of nucleotides ((e) in Figures 1 to 3) from the nucleotide adjacent 3' to the most 3' nucleotide of the "arbitrary nucleotide sequence" to be deleted, inserted, or substituted (portion (c) in Figures 1 to 3) to the nucleotide at the 3' end of the "arbitrary sense strand nucleotide sequence in genomic DNA" (portion (a) in Figures 1 to 3)
is, for example, about 10 to 1000, preferably 20 to 1000, more preferably 30 to 500, further preferably 40 to 300, further more preferably 40 to 100, and particularly preferably 40 to 70.

The ratio (the number of nucleotides in portion (e)/the number of nucleotides in portion (d)) of the number of nucleotides ((e) in Figures 1 to 3) from the nucleotide adjacent 3' to the most 3' nucleotide of the "arbitrary nucleotide sequence" to be deleted, inserted, or substituted (portion (c) in Figures 1 to 3) to the nucleotide at the 3' end of the "arbitrary sense strand nucleotide sequence in genomic DNA" (portion (a) in Figures 1 to 3)
to the number of nucleotides ((d) in Figures 1 to 3) from the nucleotide at the 5' end of the "arbitrary sense strand nucleotide sequence in genomic DNA" (portion (a) in Figures 1 to 3) to the nucleotide adjacent 5' to the most 5' nucleotide of the "arbitrary nucleotide sequence" to be deleted, inserted, or substituted (portion (c) in Figures 1 to 3)
is not particularly limited, and is for example, 0.01 to 100, preferably 0.1 to 10, more preferably 0.2 to 5, and further preferably 0.5 to 2.

The single strand polynucleotide according to the present invention includes a nucleotide sequence having 90% or more identity with the "modified nucleotide sequence" (except the same nucleotide sequence as the sense strand nucleotide sequence). The identity is preferably 95% or more, more preferably 97% or more, further preferably 99% or more, further more preferably 99.5% or more, and particularly preferably 99.9% or more.

From the viewpoint of mechanism of the aforementioned genome editing according to the present invention, the single strand polynucleotide according to the present invention is considered to interact (for example, hybridize) with genomic DNA to cause genome editing. From this viewpoint, the single strand polynucleotide according to the present invention ((b) in Figures 1 to 3) is preferably capable of interacting (for example, hybridizing) with the "arbitrary sense strand nucleotide sequence in genomic DNA" (portion (a) in Figures 1 to 3) in the cell subjected to genome editing. Specifically, the single strand polynucleotide can, for example, hybridize with the sequence under stringent conditions. The stringent conditions can be determined based on the melting temperature (Tm) of the nucleic acid that binds to a probe, as taught in Berger and Kimmel (1987, Guide to Molecular Cloning Techniques Methods in Enzymology, Vol. 152, Academic Press, San Diego CA)). Examples of washing conditions after hybridization usually include conditions such as "1 x SSC, 0.1% SDS, 37°C." The single strand polynucleotide according to the present invention is preferably one that can maintain the state of hybridization even when washed under such conditions. The conditions are not particularly limited, and examples of more stringent hybridization conditions include washing conditions such as "0.5 x SSC, 0.1% SDS, 42°C" and further stringent hybridization conditions include washing conditions such as "0.1 x SSC, 0.1% SDS, 65°C."

The single strand polynucleotide according to the present invention is typically DNA or RNA, and is preferably RNA from the viewpoint of capable of more strongly interacting (for example, hybridizing) genomic DNA.

The single strand polynucleotide according to the present invention may be modified by known chemical modification as illustrated in the following, as long as its interaction with genomic DNA is not deteriorated markedly. To prevent the degradation with hydrolases such as nucleases, the phosphoric acid residue (phosphate) in each nucleotide can be substituted with for example, a chemically modified phosphoric acid residue such as phosphorothioate (PS), methylphosphonate, or phosphorodithionate. Moreover, the hydroxyl group at the second position of the sugar (ribose) in each ribonucleotide may be substituted with -OR (R represents, for example, CH3 (2'-O-Me), CH2CH2OCH3 (2'-O-MOE), CH2CH2NHC(NH)NH2, CH2CONHCH3, CH2CH2CN, or the like). Furthermore, the base moiety (pyrimidine, purine) may be chemically modified and examples thereof include introduction of a methyl group and a cationic functional group at position 5 of the pyrimidine base, substitution of the carbonyl group at position 2 with thiocarbonyl. Furthermore, examples of the single strand polynucleotide include, but not limited to, those in which the phosphate moiety or the hydroxyl moiety is modified with, for example, biotin, an amino group, a lower alkyl amine group, and an acetyl group. Moreover, BNA (LNA), in which the conformation of the sugar moiety is fixed to N-type by cross-linking 2' oxygen and 4' carbon of the sugar moiety in the nucleotide, or the like may also be preferably used.

The single strand polynucleotide according to the present invention can readily be produced according to a known genetic engineering procedure. For example, it can be produced by utilizing PCR, restriction enzyme cleavage, a DNA ligation technique, an in vitro
transcription/translation technique.

### 2. Expression vector

The expression vector according to the present invention is an expression vector containing an expression cassette of the single strand polynucleotide according to the present invention.

The expression cassette is not particularly limited, as long as it is a polynucleotide capable of expressing (transcribing) the single strand polynucleotide according to the present invention in a cell to be modified by genome editing or cells in an organism to be modified by genome editing. Typical examples of the expression cassette include polynucleotides such as a promoter and a DNA having the coding sequence of the single strand polynucleotide according to the present invention placed under the control of the promoter.

The promoter contained in the expression cassette is not particularly limited and can be selected as appropriate according to the cell subjected to genome editing or the organism subjected to genome editing, and/or the number of nucleotides in the single strand polynucleotide according to the present invention. Examples of promoters for prokaryotic cells include the lac promoter, the tac promoter, the tet promoter, and the ara promoter. Moreover, examples of promoters for eukaryotic cells include pol II promoters such as the CMV promoter, the EF1 promoter, the SV40 promoter, the MSCV promoter, the hTERT promoter, the β actin promoter, and the CAG promoter; and pol III promoters such as the murine and human U6-snRNA promoters, the human H1-RNase P RNA promoter, and the human valine-tRNA promoter.

The expression cassette may contain other elements (for example, multicloning site (MCS)) as needed. Examples thereof include aspects in which MCS is placed on the 5' side, between the promoter and the coding sequence of the single strand polynucleotide according to the present invention (preferably adjacent to either or both of them), and/or on the 3' side of the coding sequence of the single strand polynucleotide according to the present invention (preferably adjacent to the sequence), in the expression cassette. MCS is not particularly limited as long as it contains a plurality (for example, 2 to 50, preferably 2 to 20, more preferably 2 to 10) of restriction enzyme sites.

The expression vector may have other sequences other than the aforementioned expression cassette. The other sequences are not particularly limited and various known sequences that an expression vector can contain may be employed. Examples of such sequences include a replication origin and a drug-resistance gene.

Examples of the drug-resistance gene include the chloramphenicol resistance gene, the tetracycline resistance gene, the neomycin resistance gene, the erythromycin resistance gene, the spectinomycin resistance gene, the kanamycin resistance gene, the hygromycin resistance gene, and the puromycin resistance gene.

The kind of the vector is not particularly limited and examples thereof include plasmid vectors; virus vectors such as retrovirus, lentivirus, adenovirus, adeno-associated virus, herpesvirus, and Sendai virus; and agrobacterium vectors.

The expression vector according to the present invention can readily be produced according to a known genetic engineering procedure. For example, it can be produced by utilizing PCR, restriction enzyme cleavage, a DNA ligation technique, an in vitro
transcription/translation technique.

### 3. Method of editing genome, method of producing genome-edited cell or non-human organism

A method comprising the step of introducing the single strand polynucleotide according to the present invention into a cell to be modified by genome editing or a non-human organism to be modified by genome editing allows the genome editing of such cells or organisms. More specifically, when an arbitrary nucleotide sequence in genomic DNA is deleted in the single strand polynucleotide (Figure 1), the deletion of the nucleotide sequence can be occur in the genome, when an arbitrary nucleotide sequence is inserted in the single strand polynucleotide (Figure 2), the insertion of the nucleotide sequence can be occur in the genome, and when an arbitrary nucleotide sequence is substituted in the single strand polynucleotide (Figure 3), the substitution of the nucleotide sequence can be occur in the genome.

The method of introducing the single strand polynucleotide according to the present invention is not particularly limited and can be selected as appropriate according to the kind of the cell or organism to be modified. Examples of the method of introduction include microinjection, electroporation, DEAE-dextran treatment, lipofection, nanoparticle-mediated transfection, and virus-mediated nucleic acid delivery.

Since genome editing occurs in a cell or organism to be modified after a lapse of certain time after the introduction of the single strand polynucleotide according to the present invention, collecting this cell or organism allows acquisition of a genome-edited cell or non-human organism (more specifically, when the arbitrary nucleotide sequence in genomic DNA is deleted in the single strand polynucleotide (Figure 1), the deletion of the nucleotide sequence occurred in the genome, when the arbitrary nucleotide sequence is inserted in the single strand polynucleotide (Figure 2), the insertion of the nucleotide sequence occurred in the genome, and when the arbitrary nucleotide sequence is substituted in the single strand polynucleotide (Figure 3) the substitution of the nucleotide sequence occurred in the genome).

When a cell is to be modified by genome editing, all cells or organisms into which the single strand polynucleotide is to be introduced may be collected in the aforementioned collection or genome-edited cells or organisms may be harvested. The method for sorting is not particularly limited and a known method for sorting may be employed. For example, if the expression of a particular protein is turned ON/OFF by genome editing, then genome-edited cells may be sorted by using the presence or absence of the expression of the protein as an indicator.

According to the genome editing technique according to the present invention, genome editing can be achieved only by the single strand polynucleotide according to the present invention without introducing a nuclease (ZFN protein, TALEN protein, Cas protein) and/or an expression vector thereof or a guide RNA and/or an expression vector thereof. In this regard, the method of editing genome according to the present invention is a method of editing genome for non-ZFN, non-TALEN, non-CRISPR/Cas type, the method comprising no step of introducing the aforementioned nuclease and/or expression vector thereof or a guide RNA and/or an expression vector thereof.

The method of editing genome and the method of producing a genome-edited cell or non-human organism may further comprise the step of synthesizing at least one selected from the group consisting of the single strand polynucleotide according to the present invention and an expression vector thereof before the step of introducing the single strand polynucleotide according to the present invention. Moreover, the method may further comprise, before the synthesizing step, the step of designing at least one sequence selected from the group consisting of the single strand polynucleotide according to the present invention and an expression vector thereof.

In a cell or non-human organism obtained by the genome editing technique according to the present invention, no or little, if any, unintended mutation such as random truncation or addition of DNA occurs at the genome editing portion of interest. Such unintended mutation can be decreased greatly particularly in comparison with the CRISPR/Cas system.

### 4. Kit for editing genome and composition for editing genome

The single strand polynucleotide according to the present invention may be utilized as a composition for editing genome or may be utilized as a kit.

The composition for use in editing genome is not particularly limited, as long as it contains the single strand polynucleotide according to the present invention and may further comprise other components as needed. The other components are not particularly limited, as long as they are pharmaceutically acceptable components, but examples thereof include a base material, a carrier, a solvent, a dispersant, an emulsifier, a buffer, a stabilizer, an excipient, a binder, a disintegrator, a lubricant, a viscosity-increasing agent, a humectant, a coloring agent, a flavor, and a chelating agent.

The kit for editing genome is not particularly limited as long as it contains the single strand polynucleotide according to the present invention and may comprise, as appropriate, other reagents, instruments, and the like necessary for the practice of the method of editing genome according to the present invention, such as reagents for introducing nucleic acid and buffer solutions, as needed.

According to the genome editing technique according to the present invention, genome editing can be performed only with the single strand polynucleotide according to the present invention without introducing a nuclease (ZFN protein, TALEN protein, Cas protein) and/or an expression vector thereof or a guide RNA and/or an expression vector thereof. In this regard, the composition and the kit for use in editing genome according to the present invention are a composition and a kit for use in editing genome of non-ZFN, non-TALEN, non-CRISPR/Cas type, comprising no nuclease and/or expression vector thereof or a guide RNA and/or an expression vector thereof.

### Examples

Hereinafter, the present invention will be described in detail with reference to Examples, but the present invention is not limited by these Examples.

### Example 1. Genome editing with single strand polynucleotide (PART I)

Single strand DNAs consisting of nucleotide sequences (SEQ ID NOs: 1 to 6) modified from an arbitrary sense strand nucleotide sequence in the mexR gene from Pseudomonas aeruginosa by deletion of or insertion of an arbitrary nucleotide sequence in a central portion of this sequence were synthesized at a commissioned DNA synthesis company (macrogen). The numbers of nucleotides in portions of these single strand DNAs are illustrated in Table 1. Patterns and (a) to (e) in Table 1 correspond to the patterns and (a) to (e) in Figure 1 and Figure 2.

**[Table 1]**

| Name of single strand DNA | SEQ ID NO: | Patterm | Number of nucleotides in each portion | | | | |
|---|---|---|---|---|---|---|---|
| | | | (a) | (b) | (c) | (d) | (e) |
| 100R-1 | 1 | 1 | 108 | 100 | 8 | 49 | 51 |
| 120R-1 | 2 | 1 | 128 | 120 | 8 | 59 | 61 |
| 100R-2 | 3 | 1 | 108 | 100 | 8 | 50 | 50 |
| 120R-2 | 4 | 1 | 128 | 120 | 8 | 60 | 60 |
| 109R-1 | 5 | 2 | 100 | 109 | 9 | 43 | 57 |
| 119R-1 | 6 | 2 | 110 | 119 | 9 | 48 | 62 |

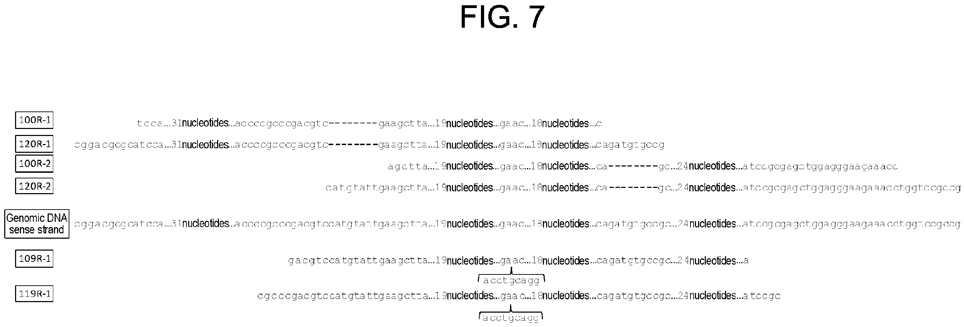

Moreover, the correspondence between the sequences of these single strand DNAs and genomic DNA is illustrated in Figure 7. In Figure 7, "-" indicates a nucleotide deleted in single strand DNAs and "}" indicates the sequence and the position inserted in genomic DNA sequence.

Since the deletion of portion (c) in Pattern 1 or the insertion of portion (c) between portion (d) and portion (e) in Pattern 2 in the mexR gene from Pseudomonas aeruginosa caused by the introduction of these single strand DNAs results in loss of the original mexR protein activity (the activity that inhibits the expression of the MexAB-OprM drug efflux pump) from the protein expressed from the gene and increases the drug-resistance of Pseudomonas aeruginosa, it is expected that the number of fungi that grow on a drug containing medium (the number of colonies) increases.

The single strand DNAs (SEQ ID NOs: 1 to 6) were each dissolved in water to 500 µM to obtain single strand DNA solutions. 1 µL of a single strand DNA solution or 1 µL of water was added to 40 µL of a solution containing competent cells of Pseudomonas aeruginosa (PAO1S-Lac) and the resulting suspension was left on ice for 20 minutes. The single strand DNA was introduced into the cells by electroporation (2500 V, 25 µF, 700 Ω) and then 960 µL of the SOC medium was added thereto. The resulting suspension was cultured with shaking at 37°C for 4 hours. The obtained culture was plated on an aztreonam-containing LB agar medium and cultured at 37°C for 2 days. After culturing, the number of colonies was counted and the relative value of the number of colonies when a single strand DNA was added to the number of colonies when water was added instead of the single strand DNA (control) (the relative colony count) was calculated. Furthermore, arbitrary 10 colonies were picked up and sequenced and the rate of colonies having the mutation of interest (the deletion of portion (c) in Pattern 1, the insertion of portion (c) between portion (d) and portion (e) in Pattern 2) was calculated. The results are illustrated in Table 2.

**[Table 2]**

| Name of single strand DNA | SEQ ID NO: | Patterm | Relative colony count | Rate of colonies having deletion or insertion of portion (c) |
|---|---|---|---|---|
| 100R-1 | 1 | 1 | 1.3 | 7/10 |
| 120R-1 | 2 | 1 | 2.4 | 6/10 |
| 100R-2 | 3 | 1 | 1.9 | 7/10 |
| 120R-2 | 4 | 1 | 2.9 | 7/10 |
| 109R-1 | 5 | 2 | 0.9 | 7/10 |
| 119R-1 | 6 | 2 | 1.5 | 5/10 |
| Control | | | 1.0 | 0/10 |

As illustrated in Figure 2, the mutation of interest (the deletion of portion (c) in Pattern 1, the insertion of portion (c) between portion (d) and portion (e) in Pattern 2) in the genome was caused by the single strand consisting of the nucleotide sequence modified from the sense strand nucleotide sequence in the genome by deletion or insertion of the arbitrary nucleotide sequence. Moreover, it was suggested from the relative colony count that increase in length of the single strand DNA increases the efficiency of introducing the mutation of interest.

The colony count of 109R-1 was equivalent to the colony count of the control (the relative colony count = 0.9). However, the result of sequencing has revealed that this was because the drug resistance was improved to some extent also in the control by spontaneous mutation (mutation (such as point mutation) different from the deletion or insertion of portion (c)). As illustrated in Figure 2, it was possible to cause the mutations of interest even when 109R-1 was used.

### Example 2. Genome editing with single strand polynucleotide (PART II)

A single strand DNA consisting of a nucleotide sequence (SEQ ID NO: 7), which has been modified from the arbitrary sense strand nucleotide sequence in the mexR gene from Pseudomonas aeruginosa by deletion of the arbitrary sequence of a central portion of this sequence, was synthesized at a commissioned DNA synthesis company (macrogen). The number of nucleotides in portions of this single strand DNA is illustrated in Table 3. Pattern and (a) to (e) in Table 3 correspond to the pattern and (a) to (e) in Figure 1 and Figure 2.

**[Table 3]**

| Name of single strand DNA | SEQ ID NO: | Pattern | Number of nucleotides in each portion | | | | |
|---|---|---|---|---|---|---|---|
| | | | (a) | (b) | (c) | (d) | (e) |
| mexRdel-100 | 7 | 1 | 506 | 100 | 406 | 49 | 51 |

The testing and sequencing were performed in the same manner as Example 1, which revealed that 2 colonies out of the 10 colonies picked up had the mutation of interest (deletion of (c)) introduced.

### Example 3. Genome editing with single strand polynucleotide (PART III)

The following single strand DNAs (A) to (D) were synthesized at a commissioned DNA synthesis company (FASMAC): (A) single strand DNAs consisting of nucleotide sequences (SEQ ID NOs: 8 to 9), which have been modified from the arbitrary sense strand nucleotide sequence in the mexT gene from Pseudomonas aeruginosa by deletion of an arbitrary nucleotide sequence in a central portion of this sequence,
(B) single strand DNAs (antisense strands of (A)) consisting of nucleotide sequences (SEQ ID NOs: 10 to 11), which have been modified from the arbitrary antisense strand nucleotide sequence in the mexT gene from Pseudomonas aeruginosa by deletion of an arbitrary nucleotide sequence in a central portion of this sequence,
(C) single strand DNAs, which is the sequences before the deletion in (A) consisting of arbitrary sense strand nucleotide sequences (SEQ ID NOs: 12 to 13) in the mexT gene from Pseudomonas aeruginosa, and
(D) single strand DNAs consisting of arbitrary antisense strand nucleotide sequences (SEQ ID NOs: 14 to 15) in the mexT gene from Pseudomonas aeruginosa, which is the sequences before the deletion in (B).

The numbers of nucleotides in portions of these single strand DNAs are illustrated in Table 4. Pattern and (a) to (e) in Table 4 correspond to the pattern and (a) to (e) in Figure 1 and Figure 2, "s" denotes a sense strand, and "a" denotes an antisense strand.

**[Table 4]**

| Name of single strand DNA | SEQ ID NO: | s / a | Patter m | Number of nucleotides in each portion | | | | |
|---|---|---|---|---|---|---|---|---|
| | | | | (a) | (b) | (c) | (d) | (e) |
| TF100 | 8 | s | 1 | 108 | 100 | 9 | 56 | 44 |
| TF120 | 9 | s | 1 | 128 | 120 | 9 | 66 | 54 |
| rTF100 | 10 | a | - | 108 | 100 | 9 | 44 | 56 |
| rTF120 | 11 | a | - | 128 | 120 | 9 | 54 | 66 |
| TF108 | 12 | s | - | 108 | 108 | - | - | - |
| TF128 | 13 | s | - | 128 | 128 | - | - | - |
| rTF108 | 14 | a | - | 108 | 108 | - | - | - |
| rTF128 | 15 | a | - | 128 | 128 | - | - | - |

Since the deletion of portion (c) in the mexT gene from Pseudomonas aeruginosa caused by the introduction of these single strand DNAs results in expression of an active form of mexT protein from the gene and thereby increases the expression of the MexEF-OprM drug efflux pump to increase the drug-resistance of Pseudomonas aeruginosa, the number of fungi (the number of colonies) that grow on a drug containing medium increases.

Tests were conducted in the same manner as Example 1 and the relative value of the number of colonies when a single strand DNA was added to the number of colonies when water was added instead of the single strand DNA (control) was calculated. The results are illustrated in Table 5 and Table 6.

**[Table 5]**

| Name of single strand DNA | SEQ ID NO: | s / a | Pattern | Relative colony count |
|---|---|---|---|---|
| TF100 | 8 | s | 1 | 4.4 |
| TF120 | 9 | s | 1 | 3.03 |
| rTF100 | 10 | a | - | 0.61 |
| rTF120 | 11 | a | - | 0.76 |
| Control | | | | 1.0 |

**[Table 6]**

| Name of single strand DNA | **SEQ ID** NO: | s / a | Pattern | Relative colony count |
|---|---|---|---|---|
| TF108 | 12 | s | - | 0.91 |
| TF128 | 13 | s | - | 1.1 |
| rTF108 | 14 | a | - | 0.84 |
| rTF128 | 15 | a | - | 0.69 |
| Control | | | | 1.0 |

As illustrated in Table 5 and Table 6, the relative colony counts were greatly increased only when the single strand DNAs consisting of the nucleotide sequences modified from (A) the sense strand nucleotide sequence by deletion of an arbitrary nucleotide sequence in a central portion of the sense strand nucleotide sequence were used. This suggests that the mutation of interest is caused in the genome only when the single strand DNA of (A) was used. The foregoing indicated that it is important for causing mutation of interest in the genome that the nucleotide sequence of the single strand DNA is a sequence based on a sense strand nucleotide sequence in the genome.

### Example 4. Genome editing with single strand polynucleotide (PART IV)

Single strand DNAs consisting of nucleotide sequences (SEQ ID NOs: 5, 6, and 16 to 21), which have been modified from an arbitrary sense strand nucleotide sequence in the mexR gene from Pseudomonas aeruginosa by insertion of an arbitrary nucleotide sequence in a central portion of this sequence, were synthesized at a commissioned DNA synthesis company (macrogen). These single strand DNAs are different in length, inserted nucleotide sequence, or the like. The numbers of nucleotides in portions of these single strand DNAs are illustrated in Table 7. Pattern and (a) to (e) in Table 7 correspond to the pattern and (a) to (e) in Figure 1 and Figure 2.

**[Table 7]**

| **Name of single strand DNA** | **SEQ ID NO:** | **Pattern** | **Number of nucleotides in each portion** | | | | |
|---|---|---|---|---|---|---|---|
| | | | (a) | (b) | (c) | (d) | (e) |
| 109R-1 | 5 | 2 | 100 | 109 | 9 | 43 | 57 |
| 109R-2 | 16 | 2 | 100 | 109 | 9 | 50 | 50 |
| 109R-4s | 17 | 2 | 100 | 109 | 9 | 50 | 50 |
| 119R-1 | 6 | 2 | 110 | 119 | 9 | 48 | 62 |
| 119R-2 | 18 | 2 | 110 | 119 | 9 | 55 | 55 |
| 119R-4s | 19 | 2 | 110 | 119 | 9 | 55 | 55 |
| 109R-3s | 20 | 2 | 100 | 109 | 9 | 50 | 50 |
| 119R-3s | 21 | 2 | 110 | 119 | 9 | 55 | 55 |

Tests were conducted in the same manner as those in Example 1. 10 colonies were picked up and PCR was conducted with a primer set located to flank the portion where the insertion is predicted using DNA extracted from each colony as template. From the obtained electrophoretic image of the PCR products, the rate of samples with which the position of the band is shifted to the higher molecular weight side (that is, samples expected to have insertion of portion (c)) was calculated. Further confirmation by sequencing was conducted and the rate of samples having insertion of portion (c)was calculated. The results are illustrated in Table 8. The rate calculated from the PCR and the rate confirmed by sequencing were almost the same.

**[Table 8]**

| Name of single strand DNA | SEQ ID NO: | Pattern | Rate of samples having insertion of portion (c) |
|---|---|---|---|
| 109R-1 | 5 | 2 | 3/10 |
| 109R-2 | 16 | 2 | 6/10 |
| 109R-4s | 17 | 2 | 9/10 |
| 119R-1 | 6 | 2 | 8/10 |
| 119R-2 | 18 | 2 | 8/10 |
| 119R-4s | 19 | 2 | 8/10 |
| 109R-3s | 20 | 2 | 7/10 |
| 119R-3s | 21 | 2 | 7/10 |

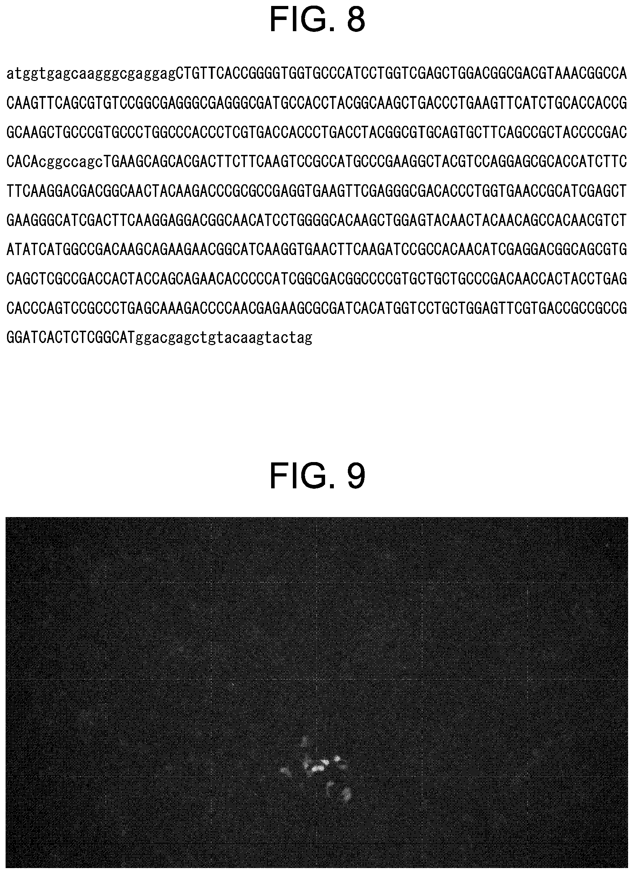

As illustrated in Figure 8, the mutation of interest (insertion of portion (c)) was caused in the genome even when different nucleotide sequences were to be inserted with the single strand DNA.

### Example 5. Genome editing with expression vector of single strand polynucleotide

The EcoR I site and the Hind III site in the MCS located downstream of the promoter in the pMMB67EH vector were cleaved with the restriction enzymes and DNA fragments containing nucleotide sequences (mexS gene deletant sequences: SEQ ID NOs: 22 to 24) modified from the full length wild type mexS gene ORF by deletion of a partial nucleotide sequence were inserted. The names of resultant vectors, SEQ ID NOs of inserted mexS gene deletant sequences, and the deletion portions in the mexS gene deletant sequences (the nucleotide at the 5' end of the wild type mexS gene ORF is designated as the first nucleotide) are shown in Table 9.

**[Table 9]**

| Name of vector | SEQ ID NO: of inserted mexS gene deletant sequence | Deletion sites in mexS gene deletant sequence |
|---|---|---|
| pMMB67EH-mexSdel26 | 22 | Nucleotides at positions 67 to 78 from 5' end of vector |
| pMMB67EH-mexSdel143 | 23 | Nucleotides at positions 206 to 216 from 5' end of vector |
| pMMB67EH-mexSdel25 | 24 | Nucleotides at positions 401 to 408 from 5' end of vector |

Pseudomonas aeruginosa (Strain 8380) in which these vectors were introduced or wild type Pseudomonas aeruginosa (Strain 8380) was suspended in 40 µL of water and the obtained suspension was added to 5 mL of LB medium (lennox) and cultured with shaking at 37°C for 17 hours. The obtained culture was plated on chloramphenicol-containing LB agar medium and cultured at 37°C for 1 day. The number of colonies was counted and the relative value of the number of colonies when Pseudomonas aeruginosa in which a vector was introduced to the number of colonies when the wild type Pseudomonas aeruginosa was used (the relative colony count) was calculated. The relative colony counts calculated from independent experiments are shown in Table 10.

When deletion of the portions deleted in the mexS gene deletants in the vectors in the mexS gene of the Pseudomonas aeruginosa genome was caused by the introduction of these vectors, the original activity (the activity that inhibits the expression of mexT) from the protein expressed from the gene is lost, thereby increases the expression of the MexEF-OprM drug efflux pump to increase the drug-resistance of Pseudomonas aeruginosa, and therefore the number of fungi (the number of colonies) that grow on a drug containing medium increases.

**[Table 10]**

| Name of vector | SEQ ID NO: of inserted mexS gene deletant sequence | Number of deleted nucleotides in mexS gene deletant sequence | Relative colony count |
|---|---|---|---|
| pMMB67EH-mexSdel26 | 22 | 12 | 209, 401 |
| pMMB67EH-mexSdel143 | 23 | 11 | 75, 40 |
| pMMB67EH-mexSdel25 | 24 | 8 | 943, 1029 |

As illustrated in Table 10, the introduction of the vectors in which the mexS gene deletant sequence was inserted largely increased the number of colonies. Among them, the number of colonies was largely increased, particularly when the number of nucleotides deleted in the mexS gene deletant sequence is 8. This suggested that the introduction of the vectors in which the mexS gene deletant sequences were inserted causes the mutation of interest (the portion that was deleted in the mexS gene deletant in the vector in the mexS gene of the Pseudomonas aeruginosa genome) was deleted.

Furthermore, as the result of sequencing, conducted separately from that described above, based on the colonies obtained in tests similar to those described above, it was confirmed that mutation of interest had occurred in all colonies when the vectors in which mexS gene deletant sequences were inserted.

### Example 6. Genome editing with single strand polynucleotide (PART V)

HEK293T cells (assay cells) in which an expression cassette of a modified GFP modified by insertion of 8 nucleotides in the coding sequence of GFP was introduced were produced. Since the modified GFP is out of the open reading frame due to the insertion of 8 nucleotides, the protein after translation becomes an inactivated form and exhibits no fluorescence. The coding sequence (SEQ ID NO: 25) of the modified GFP is set forth in Figure 8.

The assay cells were generated as follows. A plasmid (pCDH-CMV-EGFP-nonPOD-RFP+Puro, which is randomly inserted in a chromosome of cells) was mixed with the Transfection reagent and the mixture was mixed with HEK293T and the resulting cell suspension was cultured in a medium containing 0.5 ng/µl puromycin in the medium. The cell population in which the pCDH-CMV-EGFP-nonPOD-RFP+Puro vector was inserted in the cells was concentrated. This cell population was used as assay cells.

In this Example, the single strand DNAs (100EGFPdelnonPOD: SEQ ID NO: 26 and 120EGFPdelnonPOD: SEQ ID NO: 27) consisting of the nucleotide sequences modified by deletion of the aforementioned 8 nucleotides from the modified GFP were synthesized at a commissioned DNA synthesis company (macrogen). The number of nucleotides in portions of this single strand DNA is illustrated in Table 11. Pattern and (a) to (e) in Table 11 correspond to the pattern and (a) to (e) in Figure 1.

**[Table 11]**

| **Name of single strand DNA** | **SEQ ID NO:** | **Pattern** | **Number of nucleotides in each portion** | | | | |
|---|---|---|---|---|---|---|---|
| | | | (a) | (b) | (c) | (d) | (e) |
| 100EGFPdelnonPOD | 26 | 1 | 108 | 100 | 8 | 50 | 50 |
| 120EGFPdelnonPOD | 27 | 1 | 128 | 120 | 8 | 60 | 60 |

Due to the deletion of the aforementioned 8 nucleotides in the modified GFP coding sequence in the assay cells by introduction of this single strand DNA results in the express of the normal GFP protein, it is expected that the fluorescence of the cells will be observed.

The introduction of the single strand DNA was conducted as follows.

### <Preparation of FuGENE HD Transfection Reagent>

1. Prior to use, FuGENE HD Transfection Reagent was brought back to room temperature.
2. The reagent was mixed by inversion or mixed by vortex. (when the reagent was frozen by mistake and precipitation occurred, the reagent was warmed at 37 degrees Celsius for a short time to dissolve the precipitation and then brought back to room temperature.

### <Transfection of cells and confirmation of genome editing>

1 672 µl of the opti-MEM medium adjusted to room temperature beforehand was added to a sterilized tube. 14 µg of the single strand DNA solution was added and mixed by vortex. 43 µl of FuGENE HD Transfection Reagent was added thereto and promptly mixed to make a FuGENE HD Transfection Reagent/DNA mixture.
2. The FuGENE HD Transfection Reagent/DNA mixture was incubated at room temperature for 10 minutes.
3. 325 µL of the FuGENE HD Transfection Reagent/DNA mixture was added onto the plate on which assay cells proliferate and mixed gently and the cells were returned to the incubator and cultured for 4 days, subcultured into 10 cm dishes, and further incubated for 3 days.
4. After the culture, the cells were observed with a fluorescence microscope.
   A fluorescence observation image is illustrated in Figure 9. As illustrated in Figure 9, cell populations exhibiting fluorescence were observed.
5. Cell populations exhibiting color were detected by viewing and collected, and DNA was extracted from them. The intended genome editing of interest was confirmed by nucleotide sequencing with a next-generation sequencer or by the sanger method.

### Preferred Embodiments

1. A method of editing genome, comprising the step of introducing into a cell or an organism at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA,
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
2. The method of editing genome according to item 1, comprising the step of synthesizing the at least one selected from the group consisting of the single strand polynucleotide and an expression vector thereof.
3. The method of editing genome according to item 1 or 2, further comprising the step of collecting a genome-edited cell or organism.
4. The method of editing genome according to any of items 1 to 3, further comprising the step of selecting the genome-edited cell or organism.
5. The method of editing genome according to any of items 1 to 4, which is performed without step of introducing at least one selected from the group consisting of a nuclease, a guide RNA, and an expression vector thereof.
6. The method of editing genome according to any of items 1 to 5, wherein the number of nucleotides in the modified nucleotide sequence is 80 to 1000.
7. The method of editing genome according to any of items 1 to 6, wherein the number of nucleotides in the modified nucleotide sequence is 90 to 300.
8. The method of editing genome according to any of items 1 to 7, wherein the number of nucleotides in the arbitrary nucleotide to be deleted, inserted, or substituted is 1 to 920.
9. The method of editing genome according to any of items 1 to 8, wherein the ratio of the number of nucleotides in the arbitrary nucleotide sequence to be deleted, inserted, or substituted to the number of nucleotides in the modified nucleotide sequence is 10 or less.
10. The method of editing genome according to any of items 1 to 9, wherein the ratio of the number of nucleotides from the nucleotide adjacent 3' to the most 3' nucleotide of the arbitrary nucleotide sequence to be deleted, inserted, or substituted from the nucleotide at the 3' end of the sense strand nucleotide sequence to the number of nucleotides from the nucleotide at the 5' end of the sense strand nucleotide sequence to the nucleotide adjacent 5' to the most 5' nucleotide of the arbitrary nucleotide sequence to be deleted, inserted, or substituted is 0.2 to 5.
11. The method of editing genome according to any of items 1 to 10, wherein the single strand polynucleotide is a single strand DNA or single strand RNA.
12. A composition for editing genome, comprising at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA,
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
13. A kit for editing genome, comprising at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA,
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
14. A method of producing a genome-edited cell or non-human organism, comprising the step of introducing into a cell or non-human organism at least one selected from the group consisting of a single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA,
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof.
15. A single strand polynucleotide consisting of:
   (i)
      (ia) a modified nucleotide sequence having a deletion of an arbitrary endogenous nucleotide sequence from an arbitrary nucleotide sequence in genomic DNA;
      (ib) a modified nucleotide sequence having an insertion of an arbitrary nucleotide sequence to an arbitrary nucleotide sequence in genomic DNA; or
      (ic) a modified nucleotide sequence having a substitution of an arbitrary endogenous nucleotide sequence with another nucleotide sequence in an arbitrary nucleotide sequence in genomic DNA,
   wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300.
16. An expression vector comprising an expression cassette of the single strand polynucleotide according to item 15.

## Claims

1. A method of editing genome, comprising the step of introducing into a human cell at least one selected from the group consisting of a single strand DNA consisting of:
(i) a modified nucleotide sequence from an arbitrary sense strand nucleotide sequence in human genomic DNA in the human cell, the modified nucleotide sequence being modified by
(ia) deletion of an arbitrary endogenous nucleotide sequence;
(ib) insertion of an arbitrary nucleotide sequence; or
(ic) substitution of an arbitrary endogenous nucleotide sequence with another nucleotide sequence,
wherein the length of the modified nucleotide sequence is 90 to 300,
wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof, and
wherein the step is performed without step of introducing at least one selected from the group consisting of a nuclease, a guide RNA, and an expression vector thereof.

2. The method of editing genome according to claim 1, wherein the single strand DNA consists of:
(i) a modified nucleotide sequence from an arbitrary sense strand nucleotide sequence in human genomic DNA in the human cell, the modified nucleotide sequence being modified by
(ia) deletion of an arbitrary endogenous nucleotide sequence.

3. The method of editing genome according to claim 1, wherein the single strand DNA consists of:
(i) a modified nucleotide sequence from an arbitrary sense strand nucleotide sequence in human genomic DNA in the human cell, the modified nucleotide sequence being modified by
(ib) insertion of an arbitrary nucleotide sequence.

4. The method of editing genome according to claim 1, wherein the single strand DNA consists of:
(i) a modified nucleotide sequence from an arbitrary sense strand nucleotide sequence in human genomic DNA in the human cell, the modified nucleotide sequence being modified by
(ic) substitution of an arbitrary endogenous nucleotide sequence with another nucleotide sequence.

5. The method of editing genome according to any one of claims 1 to 4, wherein the single strand DNA has a chemical modification, and the chemical modification is a modification where a hydroxyl group at the second position of ribose is substituted with -O- methyl or -O-CH₂CH₂OCH₃.

6. The method of editing genome according to any one of claims 1 to 4, wherein the single strand DNA has a chemical modification, and the chemical modification is a modification where the conformation of a sugar moiety is fixed to N-type by cross-linking 2' oxygen and 4' carbon of the sugar moiety in the nucleotide.

7. The method of editing genome according to any of claims 1 to 6, wherein the number of nucleotides in the arbitrary nucleotide to be deleted, inserted, or substituted is 1 to 920.

8. The method of editing genome according to any of claims 1 to 7, wherein the ratio of the number of nucleotides in the arbitrary nucleotide sequence to be deleted, inserted, or substituted to the number of nucleotides in the modified nucleotide sequence is 10 or less.

9. The method of editing genome according to any of claims 1 to 8, wherein the ratio of the number of nucleotides from the nucleotide adjacent 3' to the most 3' nucleotide of the arbitrary nucleotide sequence to be deleted, inserted, or substituted from the nucleotide at the 3' end of the sense strand nucleotide sequence to the number of nucleotides from the nucleotide at the 5' end of the sense strand nucleotide sequence to the nucleotide adjacent 5' to the most 5' nucleotide of the arbitrary nucleotide sequence to be deleted, inserted, or substituted is 0.2 to 5.

10. A method of producing a genome-edited human cell, consisting of the step of introducing into a human cell at least one selected from the group consisting of a single strand DNA consisting of:
(i) a modified nucleotide sequence from an arbitrary sense strand nucleotide sequence in human genomic DNA in the human cell, the modified nucleotide sequence being modified by
(ia) deletion of an arbitrary endogenous nucleotide sequence;
(ib) insertion of an arbitrary nucleotide sequence; or
(ic) substitution of an arbitrary endogenous nucleotide sequence with another nucleotide sequence,
wherein the length of the modified nucleotide sequence is 90 to 300,
wherein the number of nucleotides from the nucleotide at the 5' end of the sense strand nucleotide sequence to the nucleotide adjacent 5' to the most 5' nucleotide of the arbitrary nucleotide sequence to be deleted, inserted, or substituted and the number of nucleotides from the nucleotide adjacent 3' to the most 3' nucleotide of the arbitrary nucleotide sequence to be deleted, inserted, or substituted from the nucleotide at the 3' end of the sense strand nucleotide sequence are 40 to 300; and an expression vector thereof, and
wherein the step is performed without step of introducing at least one selected from the group consisting of a nuclease, a guide RNA, and an expression vector thereof.


SEQUENCE LISTING

<110> Nexuspiral Inc.

<120> Genome editing method

<130> PN59-9001WO

<150> JP 2016-158562
<151> 2016-08-12

<160> 27

<170> PatentIn version 3.5

<210> 1
<211> 100
<212> DNA
<213> Artificial Sequence

<220>
<223> 100R-1

<400> 1
tccagagcga gctcgattgc cagcgactcg acctgacccc gcccgacgtc gaagcttatc 60

gacgaacaac gcgggctgaa cctgcaggac ctgggacgcc 100


<210> 2
<211> 120
<212> DNA
<213> Artificial Sequence

<220>
<223> 120R-1

<400> 2
cggacgcgca tccagagcga gctcgattgc cagcgactcg acctgacccc gcccgacgtc 60

gaagcttatc gacgaacaac gcgggctgaa cctgcaggac ctgggacgcc agatgtgccg 120


<210> 3
<211> 100
<212> DNA
<213> Artificial Sequence

<220>
<223> 100R-2

<400> 3
agcttatcga cgaacaacgc gggctgaacc tgcaggacct gggacgccag cgacaaggca 60

ctgatcaccc ggaagatccg cgagctggag ggaagaaacc 100


<210> 4
<211> 120
<212> DNA
<213> Artificial Sequence

<220>

<223>  120R-2

<400>  4
catgtattga agcttatcga cgaacaacgc gggctgaacc tgcaggacct gggacgccag      60

cgacaaggca ctgatcaccc ggaagatccg cgagctggag ggaagaaacc tggtccgccg      120


<210>  5
<211>  109
<212>  DNA
<213>  Artificial Sequence

<220>
<223>  109R-1

<400>  5
gacgtccatg tattgaagct tatcgacgaa caacgcgggc tgaacctgca ggacctgcag      60

gacctgggac gccagatgtg ccgcgacaag gcactgatca cccggaaga      109


<210>  6
<211>  119
<212>  DNA
<213>  Artificial Sequence

<220>
<223>  119R-1

<400>  6
cgcccgacgt ccatgtattg aagcttatcg acgaacaacg cgggctgaac ctgcaggacc      60

tgcaggacct gggacgccag atgtgccgcg acaaggcact gatcacccgg aagatccgc      119


<210>  7
<211>  100
<212>  DNA
<213>  Artificial Sequence

<220>
<223>  mexRdel-100

<400>  7
atagttgact ggatcaacca catttacatt aggtttactc ggccaaacca ccagtgcctg      60

gccgcgcaac cgcttgagga tatttaagaa cattcttttc      100


<210>  8
<211>  100
<212>  DNA
<213>  Artificial Sequence

<220>
<223>  TF100

<400>  8
gcacgaacgc agcgtgaccc gcgccgcaga gaaactgttc ctcggccagc cggccatcag      60

cgccgcgctg tcgcgcctgc gcacgctgtt cgacgacccg      100

<210> 9
<211> 120
<212> DNA
<213> Artificial Sequence

<220>
<223> TF120

<400> 9
agaccctgat gcacgaacgc agcgtgaccc gcgccgcaga gaaactgttc ctcggccagc 60

cggccatcag cgccgcgctg tcgcgcctgc gcacgctgtt cgacgacccg ctgttcgtcc 120


<210> 10
<211> 100
<212> DNA
<213> Artificial Sequence

<220>
<223> rTF100

<400> 10
cgggtcgtcg aacagcgtgc gcaggcgcga cagcgcggcg ctgatggccg gctggccgag 60

gaacagtttc tctgcggcgc gggtcacgct gcgttcgtgc 100


<210> 11
<211> 120
<212> DNA
<213> Artificial Sequence

<220>
<223> rTF120

<400> 11
ggacgaacag cgggtcgtcg aacagcgtgc gcaggcgcga cagcgcggcg ctgatggccg 60

gctggccgag gaacagtttc tctgcggcgc gggtcacgct gcgttcgtgc atcagggtct 120


<210> 12
<211> 108
<212> DNA
<213> Artificial Sequence

<220>
<223> TF108

<400> 12
gcacgaacgc agcgtgaccc gcgccgcaga gaaactgttc ctcggccagc cggccagccg 60

gccatcagcg ccgcgctgtc gcgcctgcgc acgctgttcg acgacccg 108


<210> 13
<211> 128
<212> DNA
<213> Artificial Sequence

<220>

<223>  TF128

<400>  13
agaccctgat gcacgaacgc agcgtgaccc gcgccgcaga gaaactgttc ctcggccagc      60

cggccagccg gccatcagcg ccgcgctgtc gcgcctgcgc acgctgttcg acgacccgct      120

gttcgtcc                                                              128


<210>  14
<211>  108
<212>  DNA
<213>  Artificial Sequence

<220>
<223>  rTF108

<400>  14
cgggtcgtcg aacagcgtgc gcaggcgcga cagcgcggcg ctgatggccg gctggccggc      60

tggccgagga acagtttctc tgcggcgcgg gtcacgctgc gttcgtgc                  108


<210>  15
<211>  128
<212>  DNA
<213>  Artificial Sequence

<220>
<223>  rTF128

<400>  15
ggacgaacag cgggtcgtcg aacagcgtgc gcaggcgcga cagcgcggcg ctgatggccg      60

gctggccggc tggccgagga acagtttctc tgcggcgcgg gtcacgctgc gttcgtgcat      120

cagggtct                                                              128


<210>  16
<211>  109
<212>  DNA
<213>  Artificial Sequence

<220>
<223>  109R-2

<400>  16
gacgtccatg tattgaagct tatcgacgaa caacgcgggc tgaacctgca gtgactagcg      60

gacctgggac gccagatgtg ccgcgacaag gcactgatca cccggaaga                 109


<210>  17
<211>  109
<212>  DNA
<213>  Artificial Sequence

<220>
<223>  109R-4s

<400>  17

ccgacgtcca tgtattgaag cttatcgacg aacaacgcgg gctgaacctg tgacgcgatc 60

aggacctggg acgccagatg tgccgcgaca aggcactgat cacccggaa 109

<210> 18
<211> 119
<212> DNA
<213> Artificial Sequence

<220>
<223> 119R-2

<400> 18
cgcccgacgt ccatgtattg aagcttatcg acgaacaacg cgggctgaac ctgcagtgac 60

tagcggacct gggacgccag atgtgccgcg acaaggcact gatcacccgg aagatccgc 119

<210> 19
<211> 119
<212> DNA
<213> Artificial Sequence

<220>
<223> 119R-4s

<400> 19
cccgcccgac gtccatgtat tgaagcttat cgacgaacaa cgcgggctga acctgtgacg 60

cgatcaggac ctgggacgcc agatgtgccg cgacaaggca ctgatcaccc ggaagatcc 119

<210> 20
<211> 109
<212> DNA
<213> Artificial Sequence

<220>
<223> 109R-3s

<400> 20
cccgcccgac gtccatgtat tgaagcttat cgacgaacaa cgcgggctga cgtgactaga 60

cctgcaggac ctgggacgcc agatgtgccg cgacaaggca ctgatcacc 109

<210> 21
<211> 119
<212> DNA
<213> Artificial Sequence

<220>
<223> 119R-3s

<400> 21
ctgaccccgc ccgacgtcca tgtattgaag cttatcgacg aacaacgcgg gctgacgtga 60

ctagacctgc aggacctggg acgccagatg tgccgcgaca aggcactgat cacccggaa 119

<210> 22
<211> 1008

<212>  DNA
<213>  Artificial Sequence

<220>
<223>  mexSdel26

<400>  22
atgtcccgag tgatccgttt tcatcagttt ggcccgccag aggtcctcaa atgcgaagag      60

ctgccggccg caggggaagt cctggtgcgt gtccaggcga tcggcgtgag ctggaaggat     120

gtgctctggc gtcagaacct ggccccggag caggctgcgc tgccgtccgg tctcggcttc     180

gaactggccg gcgaggtgct ggcggtcggc gccggcgtcg gcgacctgcc gctgggttcc     240

cgcgtggcca gtttccccgc ccataccccc gatcattatc cggcctatgg cgacgtggtg     300

ctgatgccgc gcgcggccct ggcggtctac cccgaggtac tcaccccggt ggaggccagc     360

gtctactaca ccggcctgct ggtggcctat ttcggcctgg tcgacctggc cgggttgaag     420

gccgggcaga ccgtgctgat caccgaggcg gcgcgcatgt acggaccggt ctcgatccag     480

ttggccaagg ctctcggcgc gcgggtgatc gcttccacca agtccgccga ggagcgcgag     540

ttcctccgcg agcagggcgc cgacaaggtg gtggtgaccg acgagcagga cctggtcctg     600

gaagtcgagc gcttcaccga gggcaagggc gtcaatgtca tcctcgacga attgggcggc     660

ccgcagatga ccctgctcgg cgatgtctcc gccacccgcg gcaagctggt gctgtatggc     720

tgcaacggcg gcaacgagtc ggcgttcccg gcctgcgccg cgttcaagaa gcacctgcag     780

ttctaccgcc actgcctgat ggatttcacc ggtcatccgg agatgggcct ggaacgcaac     840

gacgagtcgg tgagcaaggc cctcgcgcac atcgagcaac tgacccgcga tcgcctgctc     900

aagccggtgg tcgaccgggt attcgagttc gaccaggtgg tcgaggcgca ccgctacatg     960

gaaacctgtc cgaagcgcgg ccgggtggta atccacgtcg ccgattga                 1008


<210>  23
<211>  1009
<212>  DNA
<213>  Artificial Sequence

<220>
<223>  mexSdel143

<400>  23
atgtcccgag tgatccgttt tcatcagttt ggcccgccag aggtcctcaa atgcgaagag      60

ctgccgactc cggcgccggc cgcaggggaa gtcctggtgc gtgtccaggc gatcggcgtg     120

agctggaagg atgtgctctg gcgtcagaac ctggccccgg agcaggctgc gctgccgtcc     180

ggtctcggct tcgaactggc cggcggtcgg cgccggcgtc ggcgacctgc cgctgggttc     240

ccgcgtggcc agtttccccg cccatacccc cgatcattat ccggcctatg gcgacgtggt     300

gctgatgccg cgcgcggccc tggcggtcta ccccgaggta ctcaccccgg tggaggccag     360

cgtctactac accggcctgc tggtggccta tttcggcctg gtcgacctgg ccgggttgaa 420

ggccgggcag accgtgctga tcaccgaggc ggcgcgcatg tacggaccgg tctcgatcca 480

gttggccaag gctctcggcg cgcgggtgat cgcttccacc aagtccgccg aggagcgcga 540

gttcctccgc gagcagggcg ccgacaaggt ggtggtgacc gacgagcagg acctggtcct 600

ggaagtcgag cgcttcaccg agggcaaggg cgtcaatgtc atcctcgacg aattgggcgg 660

cccgcagatg accctgctcg gcgatgtctc cgccacccgc ggcaagctgg tgctgtatgg 720

ctgcaacggc ggcaacgagt cggcgttccc ggcctgcgcc gcgttcaaga agcacctgca 780

gttctaccgc cactgcctga tggatttcac cggtcatccg gagatgggcc tggaacgcaa 840

cgacgagtcg gtgagcaagg ccctcgcgca catcgagcaa ctgacccgcg atcgcctgct 900

caagccggtg gtcgaccggg tattcgagtt cgaccaggtg gtcgaggcgc accgctacat 960

ggaaacctgt ccgaagcgcg gccgggtggt aatccacgtc gccgattga 1009

<210> 24
<211> 1012
<212> DNA
<213> Artificial Sequence

<220>
<223> mexSdel25

<400> 24
atgtcccgag tgatccgttt tcatcagttt ggcccgccag aggtcctcaa atgcgaagag 60

ctgccgactc cggcgccggc cgcaggggaa gtcctggtgc gtgtccaggc gatcggcgtg 120

agctggaagg atgtgctctg gcgtcagaac ctggccccgg agcaggctgc gctgccgtcc 180

ggtctcggct tcgaactggc cggcgaggtg ctggcggtcg gcgccggcgt cggcgacctg 240

ccgctgggtt cccgcgtggc cagtttcccc gcccataccc ccgatcatta tccggcctat 300

ggcgacgtgg tgctgatgcc gcgcgcggcc ctggcggtct accccgaggt actcaccccg 360

gtggaggcca gcgtctacta caccggcctg ctggtggcct ctggtcgacc tggccgggtt 420

gaaggccggg cagaccgtgc tgatcaccga ggcggcgcgc atgtacggac cggtctcgat 480

ccagttggcc aaggctctcg gcgcgcgggt gatcgcttcc accaagtccg ccgaggagcg 540

cgagttcctc cgcgagcagg gcgccgacaa ggtggtggtg accgacgagc aggacctggt 600

cctggaagtc gagcgcttca ccgagggcaa gggcgtcaat gtcatcctcg acgaattggg 660

cggcccgcag atgaccctgc tcggcgatgt ctccgccacc cgcggcaagc tggtgctgta 720

tggctgcaac ggcggcaacg agtcggcgtt cccggcctgc gccgcgttca agaagcacct 780

gcagttctac cgccactgcc tgatggattt caccggtcat ccggagatgg gcctggaacg 840

caacgacgag tcggtgagca aggccctcgc gcacatcgag caactgaccc gcgatcgcct 900

gctcaagccg gtggtcgacc gggtattcga gttcgaccag gtggtcgagg cgcaccgcta 960

catggaaacc tgtccgaagc gcggccgggt ggtaatccac gtcgccgatt ga          1012

<210>  25
<211>  731
<212>  DNA
<213>  Artificial Sequence

<220>
<223>  modified GFP code sequence

<400>  25
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacacggcc     240

agctgaagca gcacgacttc ttcaagtccg ccatgcccga aggctacgtc caggagcgca     300

ccatcttctt caaggacgac ggcaactaca agacccgcgc cgaggtgaag ttcgagggcg     360

acaccctggt gaaccgcatc gagctgaagg gcatcgactt caaggaggac ggcaacatcc     420

tggggcacaa gctggagtac aactacaaca gccacaacgt ctatatcatg gccgacaagc     480

agaagaacgg catcaaggtg aacttcaaga tccgccacaa catcgaggac ggcagcgtgc     540

agctcgccga ccactaccag cagaacaccc ccatcggcga cggccccgtg ctgctgcccg     600

acaaccacta cctgagcacc cagtccgccc tgagcaaaga ccccaacgag aagcgcgatc     660

acatggtcct gctggagttc gtgaccgccg ccgggatcac tctcggcatg gacgagctgt     720

acaagtacta g                                                          731

<210>  26
<211>  100
<212>  DNA
<213>  Artificial Sequence

<220>
<223>  100EGFPdelnonPOD

<400>  26
gaccaccctg acctacggcg tgcagtgctt cagccgctac cccgaccaca tgaagcagca      60

cgacttcttc aagtccgcca tgcccgaagg ctacgtccag                          100

<210>  27
<211>  120
<212>  DNA
<213>  Artificial Sequence

<220>
<223>  120EGFPdelnonPOD

<400>  27

ccaccctcgt gaccaccctg acctacggcg tgcagtgctt cagccgctac cccgaccaca 60

tgaagcagca cgacttcttc aagtccgcca tgcccgaagg ctacgtccag gagcgcacca 120